# EUROPÄISCHE PATENTANMELDUNG

(11) **EP 1 044 696 A2**
(43) Veröffentlichungstag der Anmeldung: **18.10.2000**
(21) Anmeldenummer: 00107784.1
(22) Anmeldetag: 11.04.2000
(51) Int. Cl.: A61M 1/34, C12S 3/22

(54) **Entfernung von hüllehaltigen Viren aus Blut, Plasma oder Serum**

(30) Priorität: 12.04.1999 DE 19916352
(71) Anmelder: B. BRAUN MELSUNGEN AG, 34212 Melsungen (DE)
(72) Erfinder: Schettler, Volker, 37085 Göttingen (DE); Müller, Gerhard A., 70276 Tübingen (DE)
(74) Vertreter: Böhm, Brigitte, Dipl.-Chem. Dr.

(57) **Zusammenfassung**

Erfindungsgemäß wird zur Entfernung von hüllehaltigen Viren aus Blut, Plasma oder Serum ein Mittel enthaltend ein Polyanion, einen Präzipitatfilter und ein Polyanion-bindendes Adsorptionsmaterial verwendet. Damit können insbesondere HC-Viren vorteilhaft im extrakorporalen Blutkreislauf entfernt werden.

## Beschreibung

Die vorliegende Erfindung betrifft die Verwendung eines Mittels enthaltend mindestens ein Polyanion, einen Präzipitatfilter und ein Polyanionenbindendes Adsorptionsmaterial zur Entfernung von hüllehaltigen Viren aus Blut, Plasma oder Serum sowie die Verwendung eines Polyanions, eines Präzipitatfilters und eines Polyanion-bindenden Adsorptionsmaterials zur Herstellung eines Mittels zur Entfernung von hüllehaltigen Viren aus Blut, Plasma oder Serum von Patienten in einem extrakorporalen Blutkreislauf.

Die Behandlung von Virusinfektionen beim Menschen ist nach wie vor schwierig und, obwohl das Immunsystem eine Vielzahl von Viren ausreichend bekämpfen kann und bei vielen Virusinfektionen auch keine generell lebensbedrohlichen Erscheinungsformen auftreten, so existieren doch auch sehr gefährliche Viren, die zu lebensbedrohlichen Krankheiten beim infizierten Patienten führen. Die Entwicklung antiviraler Chemotherapeutika verlief langsam und war schwieriger als es bei der Entwicklung wirksamer Substanzen gegen Mikroorganismen der Fall war. Dies beruht in erster Linie auf der Tatsache, daß Viren intrazelluläre Parasiten sind, welche für die Vermehrung die aktive Beteiligung der metabolischen Prozesse von befallenen Zellen ausnützen. Deshalb ist die Verabreichung von Substanzen, die in der Lage sind, Viren abzutöten, immer auch mit der Gefahr verbunden, daß die Zellen des Wirtsorganismus geschädigt werden. Obwohl bereits seit langer Zeit versucht wird, antiviral wirkende Substanzen zu entwickeln oder aufzufinden, gibt es derzeit nur wenige zugelassene, wirksame Verbindungen. Darüber hinaus sind diese Substanzen auch nur begrenzt bei ganz bestimmten Viren wirksam. Häufig sind Behandlungen mit solchen virustatischen Substanzen mit erheblichen Nebenwirkungen für den Patienten verbunden. Teilweise können die Nebenwirkungen ebenfalls lebensbedrohliche Zustände hervorrufen. Schließlich ist die Behandlung mit derartigen antiviral wirksamen Substanzen, zu denen neben den Interferonen auch Replikationshemmer, wie Aciclovir oder Idoxoridin, sowie die Virushülle auflösende Substanzen, wie Amantitadin, gehören, teuer und, wie oben bereits ausgeführt ist, ihre Wirksamkeit und Anwendbarkeit begrenzt.

Es bestand daher ein großes Bedürfnis und damit die Aufgabe, Möglichkeiten bereitzustellen, Patienten zu behandeln, welche mit Viren, wie z.B. Hepatitis C-Virus (HC-Virus) oder HI-Virus, infiziert sind. Derartige Behandlungsmöglichkeiten sollten möglichst gut verträglich und möglichst sicher anwendbar sein.

Gelöst wird diese Aufgabe erfindungsgemäß durch die Verwendung eines Mittels enthaltend mindestens ein Polyanion, einen Präzipitatfilter und ein Polyanion-bindendes Adsorptionsmaterial zur Entfernung von hüllehaltigen Viren aus Blut, Plasma oder Serum.

Im Rahmen der vorliegenden Erfindung wurde festgestellt, daß überraschenderweise ein Verfahren, welches bisher insbesondere zur Entfernung von LDL-Cholesterin aus Blut angewandt wurde, sich auch hervorragend zur Entfernung von hüllehaltigen Viren aus dem Blut, Plasma oder Serum von Patienten eignet. Bei dem Verfahren zur Entfernung von LDL-Cholesterin handelt es sich um das sogenannte H.E.L.P.-Verfahren, welches von Seidel und Wieland entwickelt wurde (Seidel und Wieland (1982) J. Clin. Chem. Clin. Biochem. 20, 684-685; Wieland und Seidel (1983) J. Lipid. Res. 24, 904-909) und bezüglich der Entfernung von LDL aus Blut, Serum oder Plasma bereits Gegenstand einer Vielzahl von gewerblichen Schutzrechten ist. Das erfindungsgemäße Verfahren zur Entfernung von hüllehaltigen Viren kann im Prinzip analog zu dem beschriebenen H.E.L.P.-Verfahren und in analogen Apparaturen durchgeführt werden. Das H.E.L.P.-Verfahren, welches beispielsweise in der EP 0 074 610, der EP 0 166 325, der EP 0 174 478 sowie der EP 0 180 720 beschrieben ist, basiert auf den folgenden Prinzipien: Es wird einem Patienten Blut aus einem Shunt am Arm entnommen und in der Regel das Plasma von den Zellen über einen Plasmafilter getrennt. Das so gewonnene Plasma gelangt in eine Präzipitationskammer, wo es mit einem Heparinacetatpuffer vermischt wird und es zur Vernetzung und Ausfällung von LDL als LDL-Heparin-Komplex kommt. Die präzipitierten LDL-Heparin-Komplexe werden über einen Präzipitatfilter aus dem Plasma entfernt. Anschließend wird das LDL-freie Plasma-Acetatpuffer-Gemisch über einen Heparinadsorber, vorzugsweise einen lonenaustauscher, geleitet. Dort wird überschüssiges Heparin entfernt und in der Regel erfolgt anschließend noch eine Bicarbonatdialyse sowie eine Ultrafiltration des verdünnten Plasmas, um den physiologischen Blut-pH-Wert und das ursprüngliche Volumen wieder herzustellen. Am Ende des extrakorporalen Kreislaufs wird das so behandelte Plasma wieder mit den im Plasmafilter abgetrennten Blutzellen vermischt und das Vollblut dem Patienten über den venösen Teil des Shunt zurückgegeben (Eisenhower et al. (1987) Clin. Wschr. 65, 161-168).

Prinzipiell kann das H.E.L.P.-Verfahren im Rahmen der vorliegenden Erfindung in der beschriebenen Weise angewandt werden, es können jedoch auch Modifikationen, welche für die LDL-Entfernung bereits beschrieben oder/und angewandt wurden, für die Entfernung von hüllehaltigen Viren angewandt werden. Insbesondere wird hiermit auf die Offenbarung der oben genannten Patente zum grundlegenden H.E.L.P.-Verfahren Bezug genommen und diese Offenbarung zum Gegenstand der vorliegenden Anmeldung gemacht.

Das erfindungsgemäße Verfahren zur Entfernung von hüllehaltigen Viren aus Blut, Plasma oder Serum kann analog zum H.E.L.P.-Verfahren als extrakorporales Aphereseverfahren durchgeführt werden, bei dem einem Patienten entnommenes Blut im extrakorporalen Blutkreislauf behandelt wird und unmittelbar danach dem Patienten rückgeführt wird.

Es ist aber genauso denkbar, daß die Entfernung von hüllehaltigen Viren an Spenderblut durchgeführt wird, welches nach gegebenenfalls nötigen weiteren Behandlungen in einer Blutbank verwahrt wird, bis es benötigt wird. Bei dieser Anwendung ist es ebenfalls äußerst wichtig, daß gerade hüllehaltige Viren, wie Hepatitis-Viren, aus dem Blut entfernt werden, da Personen, die Fremdblut erhalten, normalerweise bereits vorgeschwächt sind und die Verabreichung von virushaltigem Blut bei ihnen besonders drastische Auswirkungen haben kann. Bei der Entfernung von Viren aus Blut, Plasma oder Serum, welches nicht direkt dem Patienten rückgeführt wird, ist es natürlich nicht unbedingt nötig, eine Aphereseapparatur zu verwenden. Es kann vielmehr in einer Umgebung gearbeitet werden, wie sie für die Behandlung von Blut im allgemeinen geeignet sind, und dabei das Prinzip des H.E.L.P.-Verfahrens benutzt werden. Bei der erfindungsgemäßen Anwendung des LDL-Aphereseverfahrens H.E.L.P. können hüllenartige Viren sehr effizient entfernt werden, so daß Spenderblut praktisch vollständig von hüllehaltigen Viren gereinigt werden kann. Auch bei einem Aphereseverfahren, bei dem das Blut nach der Behandlung dem Patienten direkt zurückgeführt wird, kann anschließend eine stark verminderte Infektiosität des betroffenen Patienten gezeigt werden. Es ist aus medizinischer Sicht denkbar, daß durch die alleinige Behandlung mit diesem H.E.L.P.-Verfahren der betroffene Patient virenfrei therapiert werden kann, wobei gegebenenfalls mehrfache Anwendung des H.E.L.P.-Verfahrens notwendig sein kann. Zusätzliche immunmodulierende Therapieformen können gegebenenfalls für den jeweiligen Viruserreger notwendig bleiben. Dies könnte insbesondere für mit HIV infizierte Personen zutreffen.

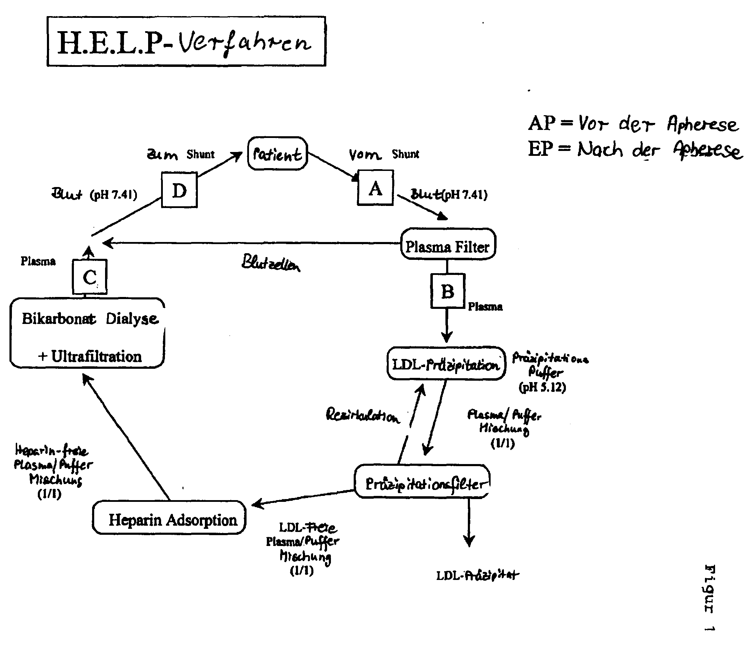

Eine schematische Darstellung des H.E.L.P.-Verfahren ist in Figur 1 gezeigt. Man kann darin die einzelnen Verfahrensschritte sehr gut erkennen, wobei die erfindungsgemäße Verwendung nicht auf die in der Figur angegebenen Schritte und Bedingungen begrenzt sein soll. Figur 1 dient aber zur Erläuterung des prinzipiell anwendbaren und kommerziell erhältlichen H.E.L.P.-Verfahrens der Firma B. Braun Melsungen AG.

Im Rahmen der vorliegenden Erfindung ist es bevorzugt, als Polyanion, wie bereits im H.E.L.P.-Verfahren, Heparin oder ein Heparinderivat zu verwenden. Obwohl im Prinzip auch andere Polyanionen im Rahmen der vorliegenden Erfindung verwendet werden können, solange sie die Fähigkeit haben, eine Ausfällung von LDL oder/und hüllehaltigen Viren in einem Präzipitationspuffer zu bewirken, so hat sich Heparin als besonders geeignet für das H.E.L.P.-Verfahren erwiesen. Allerdings sind auch Heparinderivate durchaus mit großem Erfolg anwendbar, wobei im Rahmen der vorliegenden Erfindung auch heparinartige Substanzen unter den Begriff Heparinderivat zu subsummieren sind. Solche Substanzen können beispielsweise hydrolysiertes Heparin, hydrolysiertes, an seinen freien Aminogruppen acyliertes Heparin einschließen, weitere bevorzugt verwendbare Polyanionen können jedoch auch sulfatiertes Glucosaminoglykan und andere sulfatierte Polysaccharide sein.

Im Rahmen der vorliegenden Erfindung wird das Polyanion und insbesondere das Heparin in einem Präzipitationspuffer zugesetzt, wobei man einen physiologisch verträglichen Puffer einsetzt, wobei das Verhältnis von Polyanion/Pufferlösung zu Blut, Plasma oder Serum bezüglich der Volumina zwischen 1 : 5 und 5 : 1 beträgt. Besonders vorteilhaft wird ein pH-Wert von 5,05 bis 5,25 eingestellt, bei dem die Präzipitation eines Komplexes aus Polyanionen und hüllehaltigen Viren besonders effektiv stattfinden kann. Das Präzipitat aus dem Präzipitatfilter wird verworfen, das Filtrat, welches aus Blut, Plasma oder Serum in durch Puffer verdünnter Form besteht und zusätzlich noch überschüssige Polyanionen enthalten kann, wird über das Polyanion-bindende Adsorptionsmaterial geleitet. Dabei wird überschüssiges Polyanion entfernt, in einer bevorzugten Ausführungsform der Erfindung wird noch eine Dialyse zur weiteren Reinigung des Blutes angeschlossen. Schließlich wird das Blut wieder auf die ursprüngliche Konzentration gebracht, was besonders vorteilhaft über eine Ultrafiltration erfolgt. Schließlich wird das von hüllehaltigen Viren zumindest weitgehend befreite Blut dem Patienten nach Einstellung eines physiologischen pH-Werts rückgeführt.

Die erläuterte Vorgehensweise entspricht dabei in erster Linie der besonders bevorzugten Entfernung von Viren aus dem Blut eines Patienten im extrakorporalen Blutkreislauf. Bei Entfernung von Viren aus Spenderblut, -plasma oder -serum können gegebenenfalls Abweichungen im Vorgehen erfolgen, solange die Abtrennung der hüllehaltigen Viren nicht beeinträchtigt wird. Möglich sind hier auch Kombinationen mit diagnostischen Methoden und anderen Reinigungsverfahren.

Der Präzipitatfilter weist erfindungsgemäß eine derartige Trennleistung auf, daß Komplexe aus Polyanionen und hüllehaltigen Viren sowie gegebenenfalls weiteren Substanzen, wie LDL, zurückgehalten werden, während möglichst wenige andere im Blut vorhandene und erwünschte Substanzen entfernt werden. Vorzugsweise wird ein Präzipitatfilter mit einer Porengröße von < 2µm und besonders bevorzugt mit einer Porengröße von 0,4 bis 0,8 µm verwendet. Als Filter wird dabei vorzugsweise ein Membranfilter eingesetzt. Es ist jedoch im Rahmen der vorliegenden Erfindung ebenfalls möglich, andere eine Filterwirkung aufweisende Systeme einzusetzen, solange sie ebenfalls der Bedingung genügen, daß die Komplexe aus Polyanion und hüllehaltigen Viren zurückgehalten werden, wogegen möglichst vollständig die anderen im Blut vorhandenen Substanzen das Filter passieren können.

Gegenüber bekannten Verfahren zur extrakorporalen Entfernung von unerwünschten Substanzen wie LDL aus Blut, welche statt eines Präzipitatfilters einen Adsorber verwenden, also die Substanzen mittels Adsorptionschromatographie entfernen, zeigt das erfindungsgemäße Verfahren deutliche Vorteile.

An einen Adsorber binden immer nur einzelne Moleküle, nicht bindende Substanzen durchlaufen den Adsorber unbeeinträchtigt. Je nach Art des Adsorbermaterials gelangen aber auch mehr oder weniger der eigentlich zu adsorbierenden Moleküle in das Eluat; eine vollständige Entfernung kann kaum erfolgen, da, um Verstopfungen zu vermeiden, der Adsorber ausreichend große Kanäle und Poren aufweisen muss, welche dann selbstverständlich auch von den zu entfernenden Molekülen passiert werden können, wenn diese sich nicht an eine der spezifischen Bindungsstellen im Adsorbermaterial anheften.

Im erfindungsgemäßen Verfahren wird jedoch eine andere Art der Entfernung der Viren ausgenutzt. Die hüllehaltigen Viren haben die Fähigkeit, LDL an ihrer Hülle zu adsorbieren. Vermutlich über diese Wechselwirkung mit LDL kann im Rahmen des erfindungsgemäßen Verfahrens über Heparin eine starke Vernetzung von Viren mit Heparinmolekülen stattfinden. Es bilden sich riesige Agglomerate von Heparin und hüllehaltigen Viren, diese Agglomerate werden auf dem Präzipitatfilter zurückgehalten, wodurch sich noch eine zusätzliche Schicht, eine Art Lipidmembran bildet, die wiederum kleinere Konglomerate, die möglicherweise den Präzipitatfilter noch passieren könnten, zurückhält. Dennoch konnte im Rahmen des erfindungsgemäßen Verfahrens festgestellt werden, dass trotz der außerordentlich wirksamen Rückhaltung von hüllehaltigen Viren in Komplexen mit Heparin auf den Präzipitatfiltern und der sich bildenden Konglomeratlipidschicht eine Rückhaltung von für den Patienten wichtigen Biomolekülen im Blut nur in einer solchen Weise erfolgt, dass auch nach Rückführung des Blutes für den Patienten hierdurch keine negativen Auswirkungen zu befürchten sind.

Das Polyanion-bindende Adsorptionsmaterial ist im Rahmen der Erfindung vorzugsweise ein anionenbindendes lonenaustauschermaterial. Andere Materialien, welche überschüssiges Polyanion entfernen können, sind ebenfalls im Rahmen der Erfindung dann geeignet, wenn sie keine unerwünschte Retention von im Blut vorhandenen Substanzen zeigen.

Da neben kompletten, hüllehaltigen Viren auch nicht umhüllte Virusmoleküle oder aber virale RNA im Blutstrom eines infizierten Patienten vorhanden sein können bzw. auch neue Viren von infizierten Zellen sekretiert werden können, wird in einer besonders bevorzugten Ausführungform der Erfindung dem Mittel noch ein Virustatikum zugesetzt, welches nach Rückführung des behandelten Blutes dem Patienten mitverabreicht wird.

Die Verabreichung eines Virustatikums bringt zwar möglicherweise in gewissem Umfang einige der oben stehend aufgeführten Nachteile der Behandlung von Patienten mit Chemotherapeutika mit sich, allerdings können die Dosierungen im Rahmen der vorliegenden Erfindung deutlich niedriger gehalten werden bzw. es kann kann eine viel bessere Wirksamkeit aufgrund der Entfernung der Hauptmenge der umhüllten Viren erreicht werden. Dadurch kann die Behandlungsdauer stark verkürzt werden. Noch ist nicht letztendlich geklärt, ob virale RNA oder unvollständige Viren tatsächlich infektiös sind. Eine Verabreichung von Virustatika gleichzeitig mit der Behandlung über das H.E.L.P.-Verfahren sichert jedoch den größtmöglichen Erfolg der Behandlung, sodass neben der praktisch vollständigen Entfernung von umhüllten Viren das Immunsystem zur Vernichtung der Virusteile bzw. zu einem effektiven Angriff auf neu sekretierte Viren befähigt wird. Geeignete Virustatika sind dem Fachmann bekannt, bevorzugt werden im Rahmen der vorliegenden Erfindung Interferon oder Ribavirin (1-β-D-Ribofuranosyl-1,2,4-triazol-3-carboxamid) eingesetzt.

Ein weiterer Gegenstand der vorliegenden Erfindung ist die Verwendung eines Polyanions, eines Präzipitatfilters und eines Polyanion-bindenden Adsorptionsmaterials zur Herstellung eines Mittels zur Entfernung von hüllehaltigen Viren aus Blut, Plasma oder Serum von Patienten in einem extrakorporalen Blutkreislauf. Die bevorzugten Ausgestaltungen, wie sie bereits im oben stehenden für die erfindungsgemäße Verwendung eines Mittels zur Entfernung von hüllehaltigen Viren aus Blut, Plasma oder Serum beschrieben sind, sind in gleicher Weise auf die erfindungsgemäße Herstellung eines Mittels zur Entfernung von hüllehaltigen Viren im extrakorporalen Blutkreislauf übertragbar. Insofern gelten obige Ausführungen analog.

Die erfindungsgemäßen Verwendungen ermöglichen es, in für einen Patienten schonender Weise eine gegebenenfalls sogar vollständige Entfernung von hüllehaltigen Viren aus dem Patientenblut zu bewirken. Bei Patienten mit chronischen viralen Erkrankungen, welche häufig lebenslang Virusträger sind und damit andere Personen infizieren können, wie z.B. beim Hepatitis C-Virus, kann die Behandlung zu einer zumindest stark verminderten Infektiosität dieses Personenkreises führen. Auch eine Behandlung von infizierten Personen, welche akut erkrankt sind, ist möglich und zwar ebenfalls in schonender Weise, so daß bei diesen akut erkrankten Patienten nicht noch zusätzlich oder zumindest deutlich weniger den Organismus stark belastende Arzneimittel verabreicht werden müssen. Die erfindungsgemäß verwendete Apherese kann dabei auch mehrfach hintereinander angewandt werden, so daß neu aus den Wirtszellen sezernierte Viren abgetrennt werden können und so der Kreislauf von Infektion von Zellen und Sezernierung von neu gebildeten Viren ins Blut unterbrochen werden kann.

Auch bei der Behandlung von Spenderblut kann die Gefahr, welche von chronisch infizierten Personen als Blutspendern ausgeht, weitgehend, wenn nicht sogar vollständig eliminiert werden, was hüllehaltige Viren und insbesondere das Hepatitis C-Virus betrifft.

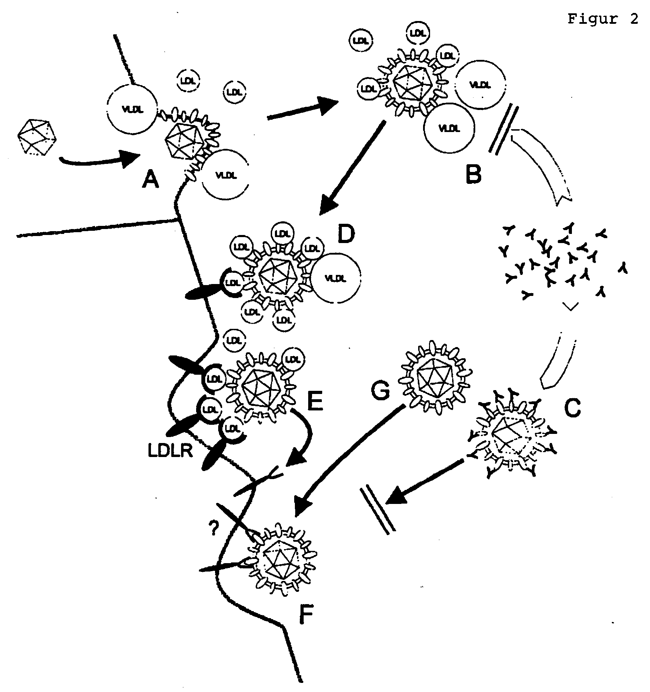

Wie oben bereits erwähnt, ist der der Erfindung zugrundeliegende Mechanismus zwar noch nicht vollständig geklärt, es wird jedoch davon ausgegangen, daß die Assoziierung von Virushüllen mit LDL hierbei eine Rolle spielt und daß möglicherweise eine Präzipitation der Komplexe über Bindung sowohl der Viren als auch von Heparin an LDL erfolgt. Eine schematische Darstellung dieses theoretischen Wirkungsmechanismus ist in Figur 2 gezeigt.

Diese Darstellung des hypothetischen Wirkungsmechanismus soll die Erfindung jedoch in keiner Weise beschränken.

Die folgenden Beispiele sollen die Erfindung weiter erläutern.

### Beispiel 1

Eine Patientin wurde nach Vorschrift mit dem H.E.L.P.-Apheresesystem der Firma B. Braun Melsungen AG (Einmalartikel: Best. Nr. 706 1102; Therapiegerät: Best. Nr. 706 2001; Katalog, Stand 1994) behandelt.

Dieser Patientin war zweimal vor der Behandlung Blut abgenommen worden, wobei sich in einer HCV-RNA-PCR positive Werte für HCV ergaben. Auch direkt vor der Apherese konnte am Behandlungstag HCV nachgewiesen werden.

Nach der Apheresebehandlung war HCV über eine PCR deutlich vermindert nachweisbar.

Die Behandlung wurde nach jeweils 3 Tagen wiederholt.
Am Ende der Behandlung konnte HCV praktisch nicht mehr nachgewiesen werden.

## Patentansprüche

1. Verwendung eines Mittels enthaltend mindestens ein Polyanion, einen Präzipitatfilter und ein Polyanion-bindendes Adsoprtionsmaterial zur Entfernung von hüllehaltigen Viren aus Blut, Plasma oder Serum.

2. Verwendung nach Anspruch 1,
**dadurch gekennzeichnet,** daß das Mittel als Polyanion Heparin oder ein Heparin-Derivat enthält.

3. Verwendung nach Anspruch 1 oder 2,
**dadurch gekennzeichnet,** daß das Mittel als Polyanion-bindendes Adsorptionsmaterial ein Ionenaustauschermaterial aufweist.

4. Verwendung nach einem der vorhergehenden Ansprüche,
**dadurch gekennzeichnet,** daß das Mittel einen Präzipitatfilter mit einer Porengröße von < 2 µm, vorzugsweise 0,4 bis 0,8 µm enthält.

5. Verwendung nach einem der vorhergehenden Ansprüche,
**dadurch gekennzeichnet,** daß als hüllehaltiges Virus Hepatitis-C-Virus entfernt wird.

6. Verwendung nach einem der vorhergehenden Ansprüche,
**dadurch gekennzeichnet,** daß die Entfernung in einem extrakorporalen Blutkreislauf stattfindet.

7. Verwendung nach einem der vorhergehenden Ansprüche,
**dadurch gekennzeichnet,** daß ein pH-Wert von 5,05 bis 5,25 eingestellt wird, bei dem ein Komplex von mindestens hüllehaltigen Viren und Polyanion ausgefällt wird und dieser Komplex anschließend über den Präzipitatfilter abgetrennt wird.

8. Verwendung nach einem der vorhergehenden Ansprüche,
**dadurch gekennzeichnet,** daß nach der Virenentfernung überschüssiges Polyanion über das Polyanion-bindende Adsorptionsmittel entfernt wird.

9. Verwendung nach Anspruch 8,
**dadurch gekennzeichnet,** daß anschließend zusätzlich eine Dialyse durchgeführt wird.

10. Verwendung nach einem der vorhergehenden Ansprüche,
**dadurch gekennzeichnet,** daß das Polyanion in einem physiologisch verträglichen Puffer gelöst vorliegt, wobei das Verhältnis von Polyanion/Puffer-Lösung zu Blut, Plasma oder Serum bezüglich der Volumina zwischen 1:5 und 5:1 liegt.

11. Verwendung nach einem der vorhergehenden Ansprüche,
**dadurch gekennzeichnet,**
daß das Mittel zusätzlich mindestens ein Virustatikum, bevorzugt Interferon oder/und Ribavirin, enthält.

12. Verwendung eines Polyanions, eines Präzipitatfilters und eines Polyanion-bindenden Adsorptionsmaterials zur Herstellung eines Mittels zur Entfernung von hüllehaltigen Viren aus Blut, Plasma oder Serum von Patienten in einem extrakorporalen Blutkreislauf.

13. Verwendung nach Anspruch 12,
**dadurch gekennzeichnet,** daß als Polyanion Heparin oder ein Heparin-Derivat verwendet wird.

14. Verwendung nach Anspruch 12 oder 13,
**dadurch gekennzeichnet,** daß als Polyanion-bindendes Adsorptionsmaterial ein lonenaustauschermaterial verwendet wird.

15. Verwendung nach einem der Ansprüche 12 bis 14,
**dadurch gekennzeichnet,** daß ein Präzipitatfilter mit einer Porengröße von < 2 µm, vorzugsweise 0,4 bis 0,8 µm verwendet wird.

16. Verfahren nach einem der Ansprüche 12 bis 15,
**dadurch gekennzeichnet,** daß ein pH-Wert von 5,05 bis 5,25 eingestellt wird, bei dem ein Komplex von mindestens hüllehaltigen Viren und Polyanion ausgefällt wird und dieser Komplex anschließend über den Präzipitatfilter abgetrennt wird.

17. Verwendung nach einem der Ansprüche 12 bis 16,
**dadurch gekennzeichnet,** daß nach der Virenentfernung überschüssiges Polyanion über das Polyanion-bindende Adsorptionsmaterial entfernt wird.

18. Verwendung nach Anspruch 17,
**dadurch gekennzeichnet,** daß anschließend zusätzlich eine Dialyse durchgeführt wird.

19. Verwendung nach einem der Ansprüche 12 bis 18,
**dadurch gekennzeichnet,** daß zusätzlich mindestens ein Virustatikum, vorzugsweise Interferon oder Ribavirin, zur Herstellung des Mittels verwendet wird.
